(19) **Europäisches Patentamt**

**European Patent Office**

**Office européen des brevets**

(11) **EP 1 017 392 B1**

(12) **EUROPÄISCHE PATENTSCHRIFT**

(45) Veröffentlichungstag und Bekanntmachung des Hinweises auf die Patenterteilung:
**17.07.2002  Patentblatt 2002/29**

(51) Int Cl.⁷: **A61K 31/495**, A61K 9/16, A61K 9/20, A61K 9/28, A61K 9/00

(21) Anmeldenummer: **98946454.0**

(22) Anmeldetag: **15.09.1998**

(86) Internationale Anmeldenummer:
**PCT/EP98/05842**

(87) Internationale Veröffentlichungsnummer:
**WO 99/15172 (01.04.1999 Gazette 1999/13)**

(54) **ARZNEIMITTELFORMULIERUNG MIT KONTROLLIERTER WIRKSTOFFFREISETZUNG**

MEDICAMENT FORMULATION WITH A CONTROLLED RELEASE OF AN ACTIVE AGENT

FORMULATION MEDICAMENTEUSE A LIBERATION CONTROLEE DU PRINCIPE ACTIF

(84) Benannte Vertragsstaaten:
**AT BE CH CY DE DK ES FI FR GB GR IE IT LI LU MC NL PT SE**
Benannte Erstreckungsstaaten:
**LT LV RO SI**

(30) Priorität: **25.09.1997  DE 19742243**

(43) Veröffentlichungstag der Anmeldung:
**12.07.2000  Patentblatt 2000/28**

(73) Patentinhaber: **Bayer Aktiengesellschaft 51368 Leverkusen (DE)**

(72) Erfinder:
 • **SIEFERT, Hans-Martin D-42105 Wuppertal (DE)**
 • **BOSCHE, Patrick D-51519 Odenthal (DE)**
 • **STASS, Heino D-51061 Köln (DE)**
 • **KETTELHOIT, Stefan D-51375 Leverkusen (DE)**
 • **LAICH, Tobias D-51065 Köln (DE)**

(56) Entgegenhaltungen:
**EP-A- 0 350 733          EP-A- 0 550 903
EP-A- 0 780 390**

 • **CHEMICAL ABSTRACTS, vol. 120, no. 26, 27. Juni 1994 Columbus, Ohio, US; abstract no. 331151, XP002090750 in der Anmeldung erwähnt & JP 06 024959 A (BAIERU YAKUHIN KK,JP) 1. Februar 1994**

## Beschreibung

Die Erfindung betrifft Arzneimittelformulierungen, die 1-Cyclopropyl-7-([S,S]-2,8-diazabicyclo[4.3.0]non-8-yl)-6-fluor-1,4-dihydro-8-methoxy-4-oxo-3-chinoloncarbonsäure (im folgenden als Verbindung I bezeichnet) und/oder pharmazeutisch verträgliche Salze davon und/oder Hydrate davon als Wirkstoff umfassen, die den enthaltenen Wirkstoff mit einer definierten Freisetzung abgeben.

Die Verbindung I besitzt den INN (International Non-Proprietary Name) Moxifloxacin.

Die Verbindung I bzw. ihre Salze und/oder Hydrate ist ein neues 8-Methoxychinolon mit antibakterieller Wirkung gegen gramnegative und grampositive Bakterien, die vielfach signifikant besser ist als diejenige von Sparfloxacin und Ciprofloxacin (Drugs of the Future 1997, 22 (2): 109/113). Die EP-A-0 305 733 sowie die EP-A-0 550 903 beschreiben die Herstellung der Verbindung I sowie ihrer pharmazeutisch verträglichen Salze. Die EP-A-0 780 390 beschreibt eine spezifische Kristallmodifikation des Monohydrat-Hydrochlorids der Verbindung I.

Tablettenformulierungen mit verzögerter bzw. kontrollierter Wirkstofffreisetzung, die Chinoloncarbonsäure-Antibiotika enthalten, sind nur wenig bekannt. Zwar wird in der JP-A-06 024 959 ein orales Arzneimittel beschrieben, das Ciprofloxacin-Hydrochlorid umfaßt, jedoch ist die Herstellung von Darreichungsformen, die den Wirkstoff über den gesamten gastrointestinalen Trakt freisetzen, für das Ciprofloxacin-Hydrochlorid praktisch nicht möglich. Der Grund dafür liegt im Absorptionsverhalten von Ciprofloxacin im Colon (S. Harder, U. Fuhr, D. Beermann, A. H. Staib, "Ciprofloxacin absorption in different regions of the human gastrointestinal tract. Investigations with the hf-capsule", Br. J. clin. Pharmac. **30,** (1990), 35-39). Die von Staib und Fuhr gefundenen Daten am Menschen bestätigen die bislang tierexperimentell vorliegenden Daten, daß Ciprofloxacin-Hydrochlorid aus dem Colon nur sehr gering absorbiert wird. Daher handelt es sich bei den weitaus meisten der bekannten Ciprofloxacin-Formulierungen mit verzögerter Wirkstofffreisetzung um nicht peroral applizierbare Arzneimittelformulierungen. So beschreibt die US-A-5473103 Ciprofloxacin-umfassende Implantate. Des weiteren beschreibt die US-A-5520920 eine Augenarzneimittelformulierung mit verzögerter Wirkstofffreisetzung. Auch für das bekannte Chinoloncarbonsäure-Antibiotikum Ofloxacin ist lediglich eine parenterale Formulierung beschrieben, die den Wirkstoff in annähernd 3 Stunden vollständig freisetzt (EP-A-0 635 272).

Die EP-A-0350733 erwähnt die Möglichkeit, die dort beschriebenen Wirkstoffe in solchen Zusammensetzungen zu formulieren, die den Wirkstoff nur oder bevorzugt in einen bestimmten Teil des Intestinaltraktes gegebenenfalls verzögert abgeben. Es werden jedoch keine konkreten Formulierungen mit verzögerter Wirkstofffreisetzung der dort offenbarten Verbindungen beschrieben. Die konkrete Tablettenformulierung, die in der EP-A-0 350 733 für die dort angegebene Verbindung des Beispiels 1 beschrieben ist, ist eine schnell freisetzende Formulierung, die den Wirkstoff in der Regel innerhalb von etwa einer halben Stunde freisetzt. Die in der EP-A-0 780 390 beschriebenen galenischen Formulierungen des Hydrochlorid-Monohydrats der Verbindung I sind ebenfalls Formulierungen mit schneller Wirkstofffreisetzung, die in der Regel zu einer Wirkstofffreisetzung innerhalb von etwa einer halben Stunde führen.

Nach Gabe einer solchen schnell freisetzenden Tablettenformulierung unterliegen die Konzentrationen des Wirkstoffs im Blut bei der für die Therapie üblichen mehrfachen Verabreichung der Arzneimittelformulierung jedoch starken Schwankungen. Nach peroraler Applikation z.B. der zuvor genannten Formulierungen mit schneller Wirkstofffreisetzung werden innerhalb von 4 Stunden die maximalen Konzentrationen des Wirkstoffs im Blut erreicht. Diese fallen dann bis zur nächsten Applikation deutlich ab. Somit ergeben sich bei mehrfacher Verabreichung von Tablettenformulierungen mit schneller Wirkstofffreisetzung starke Schwankungen der Konzentrationen des Wirkstoffs im Blut. In manchen Fällen sind jedoch hohe Konzentrationen des Wirkstoffs im Blut, die nach Verabreichung einer Tablettenformulierung mit rascher Wirkstofffreisetzung auftreten, unerwünscht, da beispielsweise auch Nebenwirkungen in verstärktem Maße auftreten können. Außerdem ist es in bestimmten Fällen wünschenswert, die Konzentrationen des Wirkstoffs im Blut auf einem höheren Niveau über einen längeren Zeitraum aufrechtzuerhalten.

Eine solche Arzneimittelformulierung mit verzögerter Freisetzung bietet darüber hinaus eine Reihe grundsätzlicher Vorteile, wie die geringere Häufigkeit der Verabreichung, die zu einer besseren Akzeptanz beim Patienten führt. Darüber hinaus können bei bestimmten Infektionen, bei denen es auf noch länger andauernde Wirkstoffspiegel als bei einer schnell freisetzenden Tablette ankommt, Vorteile erzielt werden. Insgesamt bietet eine Arzneimittelformulierung mit verzögerter Freisetzung größere Möglichkeiten, den Wirkstoffspiegel gezielt auf die spezielle Infektion und die Empfindlichkeit des Patienten abzustimmen.

Es bestand daher der Wunsch nach der Entwicklung einer Arzneimittelformulierung für die Verbindung I und deren pharmazeutisch verträgliche Salze und/oder deren Hydrate, die die zuvor beschriebenen Anforderungen erfüllt. Die Erfinder untersuchten daher zunächst intensiv das Absorptionsverhalten des Hydrochlorids der Verbindung I (im folgenden als Verbindung II bezeichnet) und fanden dabei völlig überraschend, daß, z.B. im Gegensatz zu dem oben erwähnten Ciprofloxacin, die Verbindung II auch in den tiefen Darmabschnitten (Colon, Rectum) absorbiert werden. Dieses von bekannten Chinoloncarbonsäureantibiotika verschiedene, überraschende Absorptionsverhalten des Moxifloxacins eröffnet überhaupt erst die Möglichkeit eine Retard-Formulierung des Moxifloxacins zu entwickeln.

Bei ihren weiteren intensiven Forschungen, gelang es dann auch überraschend Arzneimittelformulierungen zu entwickeln, die den Wirkstoff über einen längeren Zeitraum im gesamten Gastrointestinaltrakt freisetzen, und schließlich Arzneimittelformulierungen mit bestimmten Freisetzungsprofilen zu entwickeln, die geeignet sind, die oben beschriebenen Probleme des Stands der Technik zu überkommen.

Gegenstand der Erfindung ist somit eine Arzneimittelformulierung mit kontrollierter Wirkstoff-Freisetzung, die 1-Cyclopropyl-7-([S,S]-2,8-diazabicyclo[4.3.0]non-8-yl)-6-fluor-1,4-dihydro-8-methoxy-4-oxo-3-chinoloncarbonsäure oder pharmazeutisch verträgliche Salze und/oder Hydrate davon umfaßt, und die eine mittlere Freisetzung zwischen 80 % in 2 Stunden und 80 % in 16 Stunden und eine initiale Freisetzung von weniger als 60 % des Wirkstoffs in der ersten Stunde der Wirkstofffreisetzung aufweist.

Zur Ermittlung der initialen und mittleren Freisetzung gemäß der Definition der Erfindung werden die Arzneimittelformulierungen der vorliegenden Erfindung in der "Apparatur 2" der USP XXIII geprüft (The United States Pharmacopeia USP XXIII 1995, Seite 1791-1792). Als Testmedium wird 900 ml 0,1 molare Salzsäure oder ein Phosphatpuffer mit pH 7,4 verwendet. Die Umdrehungsgeschwindigkeit des Rührers beträgt 50 Umdrehungen pro Minute. Proben werden durch ein 8 μm Filter gezogen und deren Wirkstoffgehalt bestimmt. Die auf diese Weise als aufgelöst bestimmte Wirkstoffmenge wird in Gewichts-Prozent der eingesetzten Wirkstoffmenge umgerechnet.

Die Arzneimittelformulierung mit kontrollierter Wirkstofffreisetzung der vorliegenden Erfindung weist bevorzugt eine mittlere Freisetzung von 80 % im Zeitraum zwischen 4 und 14 Stunden (80 % in 4 Stunden und 80 % in 14 Stunden) auf.

In einer noch bevorzugteren Ausführungsform der Arzneimittelformulierung mit kontrollierter Wirkstofffreisetzung der vorliegenden Erfindung besitzt die Formulierung eine mittlere Freisetzung von 80 % im Zeitintervall zwischen 7 Stunden und 13 Stunden und eine initiale Freisetzung von weniger als 50 % des Wirkstoffs in der ersten Stunde der Freisetzung.

Die Arzneimittelformulierung mit kontrollierter Wirkstofffreisetzung der vorliegenden Erfindung läßt sich so formulieren, daß man eine relative hohe initiale Freisetzung in der ersten Stunde von 30 bis 60 % des Wirkstoffs oder eine relativ niedrige initiale Freisetzung in der ersten Stunde von 0 bis 30 % des Wirkstoffs erhält.

In einer bevorzugten Ausgestaltung der Arzneimittelformulierung mit kontrollierter Wirkstofffreisetzung mit einer relativ hohen initialen Freisetzung zwischen 45 und 55 % des Wirkstoffs in der ersten Stunde der Freisetzung weist diese eine mittlere Freisetzung von 80 % im Zeitintervall von 8 Stunden bis 12 Stunden auf.

In einer bevorzugten Ausgestaltung der Arzneimittelformulierung mit kontrollierter Freisetzung, die eine relativ niedrige initiale Freisetzung zwischen 0 und 20 % des Wirkstoffes in der ersten Stunde der Freisetzung aufweist, ist diese Formulierung durch eine mittlere Freisetzung von 80 % im Zeitraum zwischen 8 Stunden und 12 Stunden gekennzeichnet.

Die zuvor beschriebenen Arzneimittelformulierungen mit kontrollierter Wirkstofffreisetzung liegen z.B. in der Form von diffusionskontrollierten Pellets vor. Diese diffusionskontrollierten Pellets bestehen z.B. aus Neutralpellets, auf die eine Mischung des Wirkstoffs mit üblichen Binde- und Verdickungsmittel gegebenenfalls gemeinsam mit üblichen Hilfs- und Trägerstoffen, wie z.B. unten definiert, aufgetragen wird und die anschließend mit einem Diffusionslack der Weichmacher enthält, überzogen werden, oder sie bestehen aus einem wirkstoffhaltigen Kern, der mit einem Diffusionslack überzogen ist.

Als Binde- und Verdickungsmittel werden bevorzugt Hydroxypropylmethylcellulose oder Polyvinylpyrrolidon verwendet. Ebenso können andere natürliche, synthetische oder halbsynthetische Polymere wie beispielsweise Methylcellulose, Hydroxypropylcellulose, Natriumcarboxymethylcellulose, Polyacrylsäuren, Polyvinylalkohole oder Gelatine eingesetzt werden.

Als Diffusionslack eignet sich besonders Ethylcellulose, wie sie z.B. als wässrige Dispersion unter der Bezeichnung Aquacoat® oder Surelease® im Handel ist. Aber auch andere Materialien wie Acrylate (Eudragit®), Celluloseacetat, Celluloseacetatbutyrat können verwendet werden.

Als Weichmacher eignen sich beispielsweise Phthalsäurederivate (z.B. Dimethylphthalat, Diethylphthalat, Dibutylphthalat), Zitronensäurederivate (z.B. Triethylcitrat, Tributylcitrat, Acetyltriethylcitrat), andere Ester (z.B. Diethylsebacat, Triacetin), Fettsäuren und Derivate (Glycerolmonostearat, acetylierte Fettsäureglyceride, Rizinusöl und andere native Öle, Miglyol), Polyole (Glycerol, 1,2-Propandiol, Polyethylenglycol unterschiedlicher Kettenlänge). Des weiteren werden Art und Menge des Weichmachers so eingestellt, daß die oben definierte erfindungsgemäße Freisetzung und die erforderliche Stabilität der Pellets erreicht wird.

Die Einstellung der oben definierten Freisetzung erfolgt durch Steuerung der Porengröße des Diffusionslackes und seiner Dicke. Als Porenbildner zur Steuerung der Porengröße können lösliche Polymere, wie z.B. Polyethylenglycole, Polyvinylpyrolidone, Hydroxypropylmethylcellulosen, Carboxymethylcellulosen oder deren Salze, Methylcellulosen, Dextrine, Maltodextrine, Cyclodextrine, Dextrane oder andere lösliche Verbindungen, wie z.B. Salze (Kochsalz, Kaliumchlorid, Ammoniumchlorid usw.), Harnstoff, Zucker (Glucose, Saccharose, Fructose, Lactose usw.), Zuckeralkohole (Mannit, Sorbit, Lactitol usw.) eingesetzt werden. Der Anteil des Porenbildners an der Lackmenge beträgt dabei 0 bis 50 % (G/G), bevorzugt bei 0 bis 25 %, oder 5 bis 25 % (G/G) (G = Masse).

Bei den Pellets ist es besonders wichtig, ein bestimmtes Gewichts-Verhältnis von wirkstoffüberzogenen Pellets zur Diffusionsmembran sowie ein bestimmtes Verhältnis von Diffusionslack zur Weichmachermenge zu verwenden.

Teile des eingesetzten Weichmachers können während des Lackierens und Nachtemperns abdunsten. Bei Änderung der Randbedingungen ist eine Veränderung der erfindungsgemäßen Beschichtungsmenge an Diffusionlack erforderlich. So ist beispielsweise eine höhere Beschichtungsmenge erforderlich, wenn die gewünschte Freisetzungsrate verringert wird, die Menge Porenbildner erhöht wird oder bei bestimmten Weichmachern der Weichmacheranteil verringert wird. Eine niedrigere Beschichtungsmenge ist erforderlich, wenn die gewünschte Freisetzungsrate erhöht wird, die Menge Porenbildner erniedrigt wird oder bei bestimmten Weichmachern der Weichmacheranteil erhöht wird.

Die erfindungsgemäßen Diffusionspellets können beispielsweise hergestellt werden, indem man den Wirkstoff in Wasser suspendiert bzw. löst und mit einer konzentrierten Hydroxypropylmethylcellulose-Lösung verdickt. Die so erhaltene Suspension wird in einer Wirbelschichtanlage in einem Sprühprozeß auf Neutralpellets aufgezogen. Es folgt die Beschichtung der Pellets mit einer Diffusionsmembran durch Aufsprühen beispielsweise einer wäßrigen Ethylcellulosedispersion bevorzugt in einer Wirbelschichtanlage, die einen geeigneten, physiologisch verträglichen Weichmacher enthält. Die Pellets werden anschließend bei Temperaturen von 50 bis 125°C, vorzugsweise 60 bis 110°C getempert. Dabei führen höhere Temperaturen der Temperung dazu, daß zur Erzielung der erfindungsgemäßen Freisetzung eher niedere Lackauftragsmengen ausreichen und die entstehenden Pellets bei Lagerung physikalisch stabiler sind. Die Dicke der Diffusionsmembran, Weichmachertyp, Weichmachermenge und Pelletgröße werden so gewählt, daß eine Freisetzungsgeschwindigkeit von 80 % der Verbindung I oder II in 2 bis 16 Stunden resultiert und in der ersten Stunde weniger als 60 % der Dosis abgegeben wird. Die einer Tagesdosis von beispielsweise 400 mg der Verbindung I (Betain-Form) entsprechende Menge Pellets wird in eine Hartgelatinekapsel gefüllt.

Neben der beschriebenen Beschichtung von Neutralpellets sind auch andere Methoden der Pelletherstellung gangbar wie das Extrusions-/Spheronizer-Verfahren, die Rotorgranulation oder die Wirbelschichtagglomeration.

Ein Diffussionspellet besteht demnach im Falle von beschichteten Neutralpellets aus 10 bis 50 % (G/G) (G = Masse) Neutralpellets (beispielsweise Saccharose und Bindemittel oder Citronensäure), bevorzugt 10 bis 40 % (G/G) Neutralpellets, auf die 10 bis 85 % (G/G) Wirkstoffschicht, bevorzugt 30 bis 75 % (G/G), aufgebracht werden, besonders bevorzugt sind für hohe Dosierungen des Wirkstoffes 10 bis 30 % ($\geq$ 400 mg des Betains pro Einzeldosis) (G/G) Neutralpellets, auf die 50 bis 85 % (G/G) Wirkstoffschicht aufgebracht werden. Die Wirkstoffschicht besteht aus 70 bis 99.5 % (G/G) Wirkstoff and 0.5 bis 30 % (G/G) Bindemittel, bevorzugt werden dabei 80 bis 99.5 % (G/G) Wirkstoff und 0.5 bis 20 % (G/G) Bindemittel, besonders bevorzugt sind für hohe Dosierungen des Wirkstoffes 90 bis 99.5 % (G/G) Wirkstoff und 0.5 bis 10 % (G/G) Bindemittel.

Auf die so erhaltenen Pellets wird der Diffusionslack bzw. die Diffusionsschicht, bevorzugt in einer menge von 5 bis 40 % (G/G), aufgebracht, der/die, bezogen auf die Lackmenge, aus 40 bis 90 % (G/G) Filmbildner (filmbildendes Polymer z.B. Ethylcellulose (Aquacoat® oder Surelease®), Acrylate (Eudragit®), Celluloseacetat, Celluloseacetatbutyrat), bevorzugt 50 bis 85 % (G/G), besonders bevorzugt 60 bis 85 % (G/G), Porenbildner (lösliche Polymere, wie z.B. Polyethylenglycole, Polyvinylpyrolidone, Hydroxypropylmethylcellulosen, Carboxymethylcellulosen oder deren Salze, Methylcellulosen, Dextrine, Maltodextrine, Cyclodextrine, Dextrane oder andere lösliche Verbindungen, wie z.B. Salze (Kochsalz, Kaliumchlorid, Ammoniumchlorid usw.), Harnstoff, Zucker (Glucose, Saccharose, Fructose, Lactose usw.), Zuckeralkohole (Mannit, Sorbit, Lactitol usw.) im Bereich von 0 bis 50 % (G/G), bevorzugt 0 bis 35 % (G/G), besonders bevorzugt 0 bis 25 % (G/G) oder 5 bis 25 % (G/G), und Weichmacher im Bereich 5 bis 50 % (G/G), bevorzugt 5 bis 35 % (G/G), besonders bevorzugt 10 bis 35 % (G/G) besteht.

Ein Diffussionspellet besteht im Falle von beschichteten Wirkstoffpellets aus 50 bis 95 % (G/G) Wirkstoffpellets, bevorzugt 60 bis 95 % (G/G), besonders bevorzugt sind für hohe Dosierungen des Wirkstoffes ($\geq$ 400 mg des Betains pro Einzeldosis) 70 bis 95 % (G/G) Wirkstoffpellets. Diese Wirkstoffpellets bestehen aus 70 bis 99.5 % (G/G) Wirkstoff und 0.5 bis 30 % (G/G) Bindemittel, bevorzugt werden dabei 80 bis 99.5 % (G/G) Wirkstoff und 0.5 bis 20 % (G/G) Bindemittel, besonders bevorzugt sind für hohe Dosierungen des Wirkstoffes 90 bis 99.5 % (G/G) Wirkstoff und 0.5 bis 10 % (G/G) Bindemittel, sowie gegebenenfalls weiteren Zuschlagstoffe (Mikrokristalline Cellulose, thermoplastisches Polymer, andere pharmazeutisch gebräuchliche Hilfsstoffe).

Auf die beschriebenen Pellets wird der Diffusionslack bzw. die Diffusionsschicht in einer Menge von 5 bis 50 % (G/G) aufgebracht, der/die, bezogen auf die Lackmenge, aus 40 bis 90 % (G/G) Filmbildner (filmbildendes Polymer z.B. Ethylcellulose (Aquacoat® oder Surelease®), Acrylate (Eudragit®), Celluloseacetat, Celluloseacetatbutyrat), bevorzugt 50 bis 85 % (G/G), besonders bevorzugt 60 bis 85 % (G/G), Poreribildner (lösliche Polymere, wie z.B. Polyethylenglycole, Polyvinylpyrolidone, Hydroxypropylmethylcellulosen, Carboxymethylcellulosen oder deren Salze, Methylcellulosen, Dextrine, Maltodextrine, Cyclodextrine, Dextrane oder andere lösliche Verbindungen, wie z.B. Salze (Kochsalz, Kaliumchlorid, Ammoniumchlorid usw.), Harnstoff, Zucker (Glucose, Saccharose, Fructose, Lactose usw.), Zuckeralkohole (Mannit, Sorbit, Lactitol usw.) im Bereich von 0 bis 50 % (G/G), bevorzugt 0 bis 35% (G/G), besonders bevorzugt 0 bis 25 % (G/G) oder 5 bis 25 % (G/G), und Weichmacher im Bereich 5 bis 50 % (G/G), bevorzugt 5 bis 35 % (G/G), besonders bevorzugt 10 bis 35 % (G/G) besteht.

In einer weiteren Ausgestaltung der Arzneimittelformulierung mit kontrollierter Wirkstofffreisetzung der vorliegenden Erfindung werden Formulierungen verwendet, die den Wirkstoff in einer Matrix eines wasserquellbaren Polymers umfassen. Bevorzugt liegen diese Formulierungen in der Form einer Tablette vor.

Diese sogenannten Matrixformulierungen enthalten zweckmäßig von 30 bis 70 Gew.-%, bevorzugt 40 bis 60 Gew.-% des Wirkstoffs.

Der Mengenanteil der Matrix des wasserquellbaren Polymers beträgt zweckmäßig von 30 bis 50 Gew.-%, bevorzugt 30 bis 40 Gew.-%.

Außerdem bevorzugt sind erfindungsgemäße Arzneizubereitungen in Form von Erosionstabletten. Diese Tabletten sind dadurch gekennzeichnet, daß sie neben üblichen Hilfs- und Trägerstoffen sowie Tablettierhilfsstoffen, eine bestimmte Menge an wasserquellbaren, hydrogelbildenden Polymeren enthalten, wobei diese Polymere eine Viskosität von mindestens 15, bevorzugt mindestens 50 mPa • s (gemessen als 2 %ige wäßrige Lösung bei 20°C) haben müssen.

Übliche Hilfs- und Trägerstoffe sind beispielsweise Laktose, mikrokristalline Cellulose, Mannit oder Calciumphosphate. Diese liegen in einer Menge von zweckmäßig 0 bis 50 Gew.-% bevorzugt 10 bis 40 Gew.-%, besonders bevorzugt 20 bis 40 Gew.-% vor.

Übliche Tablettierhilfsmittel sind beispielsweise Magnesiumstearat, Talkum oder hochdisperses Siliciumdioxid (Aerosil®). Diese liegen im Falle des Magnesiumstearats zweckmäßig in einer Menge von 0,5 bis 1,5 Gew.-%, im Falle des hochdispersen Siliciumdioxids zweckmäßig in einer Menge von 0,1 bis 0,5 Gew.-% vor.

Als wasserlösliche, hydrogelbildende Polymere werden bevorzugt Hydroxypropylcellulosen, Hydroxypropylmethylcellulosen (HPMC), Methylcellulosen, Carboxymethylcellulose, Alginate, Galaktomannane, Polyacrylsäuren, Polymethacrylsäuren oder Copolymerisate aus Methacrylsäure und Methylmethacrylat, Guar, Agar, Pektin, Tragant, Gummi arabicum, Xanthan bzw. Mischungen dieser Substanzen eingesetzt.

Besonders bevorzugt ist die Verwendung von HPMC.

Hierbei sollten die erfindungsgemäßen Erosionstabletten bevorzugt mindestens 10 Gew.-% eines Hydroxypropylmethylcellulosetyps bezogen auf die Masse einer Tablette enthalten, dessen Viskosität (gemessen als 2 %ige wäßrige Lösung bei 20°C) mindestens 15, bevorzugt mindestens 50 mPa • s beträgt.

Die Arzneimittelformulierung, die den Wirkstoff in einer Matrix eines wasserquellbaren Polymers umfaßt, wird hergestellt, indem man den Wirkstoff, das Polymer und geeignete Hilfs- und Trägerstoffe (wie oben beschrieben) sowie übliche Tablettierhilfsmittel (wie oben beschrieben) mischt und direkt tablettiert. Ferner ist es möglich, den Wirkstoff, das wasserquellbare Polymer und geeignete Trägerstoffe in der Wirbelschicht zu granulieren. Dabei wird die Menge und Viskosität des wasserquellbaren Polymers so gewählt, daß Tabletten mit den oben beschriebenen mittleren Freisetzungsgeschwindigkeiten und initialen Freisetzungn resultieren. Das trockene Granulat wird gesiebt, mit einem Schmiermittel, wie zum Beispiel Magnesiumstearat, vermischt und tablettiert. Die Tablette wird gegebenenfalls noch lackiert.

In einer weiteren Ausführungsform der Arzneimittelformulierung mit kontrollierter Wirkstofffreisetzung der vorliegenden Erfindung handelt es sich um ein osmotisches Arzneimittelfreisetzungssystem. Solche osmotischen Arzneimittelfreisetzungssysteme sind grundsätzlich im Stand der Technik bekannt und werden z.B. ausführlich abgehandelt in Richard W. Baker, "Osmotic Drug Delivery: A Review of the Patent Literature", Journal of Controlled Release 35 (1995) 1-21. Die Arzneimittelformulierung als osmotisches Arzneimittel-Freisetzungssystem besteht bevorzugt aus

a) einem Kern, der den Wirkstoff, gegebenenfalls ein hydrophiles polymeres Quellmittel und gegebenenfalls einen wasserlöslichen Stoff zur Induzierung der Osmose enthält, und

b) einer für Wasser durchlässigen und für die Komponenten des wirkstoffhaltigen Kerns undurchlässigen Hülle

c) einer Öffnung durch die Hülle b) für den Transport der im Kern enthaltenen Bestandteile in die umgebende Körperflüssigkeit.

Dieses spezielle osmotische Arzneimittelfreisetzungssystem ist grundsätzlich im Stand der Technik beschrieben, beispielsweise in der DE-A-2 328 409 oder der US-A-3 85 770. Bezüglich der Materialien für die Hülle sei auf die EP-A-0 277 092 und die dort erwähnten US-A-3 916 899 und US-A-3 977 404 Bezug genommen.

Bezüglich geeigneter hydrophiler polymerer Quellmittel sei beispielsweise auf die in der EP-A-0 277 092 sowie der WO 96/40080 genannten polymeren Quellmittel verwiesen. Beispielsweise können Ethylenoxidhomopolymerisate (Polyethylenglycol) mit verschiedenen Polymerisationsgraden, die beispielsweise unter der Bezeichnung Polyox® bekannt sind, mit Molekulargewichten zwischen 100.000 bis 8.000.000 sowie Vinylpyrrolidon-vinylacetat-copolymerisate sowie weitere in der US-A-3 865 108, US-A-4 002 173 und US-A-4 207 893 genannte wasserquellbare Polymere verwendet werden.

Wasserlösliche Stoffe zur Induzierung der Osmose sind im Prinzip alle wasserlösliche Stoffe, deren Verwendung in der Pharmazie unbedenklich ist, die z.B. in den Pharmacopöen oder in "Hager's Handbuch der Pharmazeutischen Praxis, 1990-1995, Springer Verlag" sowie Remington's Pharmaceutical Sciences als wasserlösliche Hilfsstoffe erwähnt sind. Da die Verbindung I bzw. ihre Salze und/oder Hydrate über eine relativ hohe Wasserlöslichkeit (ca. 24 g/Liter) verfügt, ist auch der Wirkstoff selbst osmotisch wirksam. Dies wird bei der Formulierung des osmotischen Arzneimittelsystems berücksichtigt. Weitere spezielle wasserlösliche Stoffe sind Salze von anorganischen oder organischen Säuren oder nichtionische organische Stoffe mit hoher Wasserlöslichkeit wie z.B. Kohlenhydrate wie Zucker etc. Die Herstellung einer Öffnung in die Hülle der Tablette ist an sich im Stand der Technik bekannt und beispielsweise in den US Patentschriften 3 485 770 und 3 916 899 beschrieben.

Die Einstellung der oben beschriebenen mittleren Freisetzung sowie der initialen Freisetzung der Arzneimittelformulierung mit kontrollierter Wirkstofffreisetzung der vorliegenden Erfindung erfolgt durch Art und Menge des die Hülle bildenden semipermeablen Materials, durch Art und Menge des ggf. enthaltenen hydrophilen polymeren Quellmittels sowie des gegebenenfalls vorhandenen wasserlöslichen Stoffes zur Induzierung des Osmose,

Die Arzneimittelformulierungen der Erfindung enthalten zweckmäßig, bezogen auf die 1-Cyclopropyl-7-([S,S]-2,8-diazabicyclo[4.3.0]non-8-yl)-6-fluor-1,4-dihydro-8-methoxy-4-oxo-3-chinoloncarbonsäure, 200 bis 800 mg, bevorzugt 400 bis 600 mg des Wirkstoffs.

Die Arzneimittelformulierung mit kontrollierter Wirkstofffreisetzung der vorliegenden Erfindung ist bevorzugt eine solche Formulierung, bei der bei gleicher Dosismenge der maximale Blutspiegelwert ($c_{max}$) niedriger ist als der Wert $c_{max}$ einer Arzneimittelformulierung mit rascher Freisetzung, wie sie in Beispiel 7 der EP-A-0 780 390 beschrieben ist, und bei der die peak trough Fluktuation PTF [%] geringer ist als der entsprechende Wert PTF der Formulierung von Beispiel 7 gemäß EP-A-0 780 390.

$$PTF = c_{max} - c_{min} / c_{av,\tau}$$

$c_{min}$:     minimale Konzentration des Wirkstoffs im Blut, Plasma oder Serum

$c_{av,\tau}$:     mittlere steady state Konzentration berechnet aus Plasmakonzentrationszeitdaten nach Einmalapplikation oder nach dem ersten Dosisintervall ($\tau$) nach Mehrfachapplikation.

Die Bestimmung von PTF ist in H. Boxenbaum, "Pharmacokinetic determinants in the design and evaluation of sustained release dosage forms", Pharm. Res., 15, 82-88 (1984) beschrieben.

Die Bestimmung der Blutspiegelwerte erfolgt wie in H. Stass, A. Dalhoff, D. Kubitza, "BAY 12-8039, A new 8-Methoxy-Quinolone: First pharmacokinetic results in healthy male volunteeers", Proc. of 36th ICAAC, New Orleans, 1996, F024, page 104 beschrieben.

**Beispiele**

**Vergleichsbeispiel 1**

(schnell freisetzende Tablette, entsprechend Stand der Technik, gemäß Beispiel 7 der EP-A- 0 780 390):

Zusammensetzung:

| | |
|---|---|
| Verbindung II (Hydrochlorid) | 436,8 mg |
| Mikrokristalline Cellulose | 61,8 mg |
| Maisstärke | 31,8 mg |
| Croscarmellose Natrium | 3,6 mg |
| Magnesiumstearat | 8,0 mg |

Die Tablette wurde analog Beispiel 7 der EP-A-0 780 390 hergestellt.

**Beispiel 1**

(Matrixtablette):

Eine Matrixtablette ist wie folgt zusammengesetzt:

| | |
|---|---|
| Verbindung II (Hydrochlorid) | 436,8 mg |
| HPMC 90 SH 100 | 191,0 mg |

(fortgesetzt)

| | |
|---|---|
| Magnesiumstearat | 8,0 mg |
| Eisenoxid | 0,3 mg |
| Titandioxid | 2,7 mg |
| Polyethylenglykol 4000 | 3,0 mg |
| HPMC 15 cP | 9,0 mg |

Verbindung II (Hydrochlorid), HPMC 90 SH 100 und Magnesiumstearat werden trocken vermischt und zu Tabletten verpreßt. Die Tabletten werden mit einer wäßrigen Suspension, die das Eisenoxid, das Titandioxid, das Polyethylenglykol 4000 und die HPMC enthält, lackiert.

**Beispiel 2**

(Matrixtablette):

Eine Matrixtablette ist wie folgt zusammengesetzt:

| | |
|---|---|
| Verbindung II (Hydrochlorid) | 436,8 mg |
| HPMC 15 cP | 334,0 mg |
| Lactose Monohydrat | 334,0 mg |
| Magnesiumstearat | 8,2 mg |
| Eisenoxid | 0,45 mg |
| Titandioxid | 4,05 mg |
| Polyethylenglykol 4000 | 4,5 mg |
| HPMC 15 cP | 13,5 mg |

Verbindung II (Hydrochlorid), HPMC 15 cP und Lactose werden in einem Wirbelschichtgranulator granuliert. Das Magnesiumstearat wird zugemischt und diese Mischung zu Tabletten verpreßt. Die Tabletten werden mit einer wäßrigen Suspension, die das Eisenoxid, das Titandioxid, das Polyethylenglykol 4000 und die HPMC enthält, lackiert.

**Beispiel 3**

(Matrixtablette):

Eine Matrixtablette ist wie folgt zusammengesetzt:

| | |
|---|---|
| Verbindung II (Hydrochlorid) | 436,8 mg |
| HPMC 15 cP | 334,0 mg |
| Calciumhydrogenphosphat | 334,0 mg |
| Magnesiumstearat | 8,2 mg |
| Eisenoxid | 0,45 mg |
| Titandioxid | 4,05 mg |
| Polyethylenglykol 4000 | 4,5 mg |
| HPMC 15 cP | 13,5 mg |

Verbindung II (Hydrochlorid), HPMC 15 cP und Calciumhydrogenphosphat werden granuliert. Das Magnesiumstearat wird zugemischt und diese Mischung zu Tabletten verpreßt. Die Tabletten werden mit einer wäßrigen Suspension, die das Eisenoxid, das Titandioxid, das Polyethylenglykol 4000 und die HPMC enthält, lackiert.

**Beispiel 4**

(Matrixtablette):

Eine Matrixtablette ist wie folgt zusammengesetzt:

| Verbindung II (Hydrochlorid) mikronisiert | 436,8 mg |
|---|---|
| HPMC 50 cP | 109,2 mg |
| Magnesiumstearat | 4,0 mg |
| Eisenoxid | 0,3 mg |
| Titandioxid | 2,7 mg |
| Polyethylenglykol 4000 | 3,0 mg |
| HPMC 15 cP | 9,0 mg |

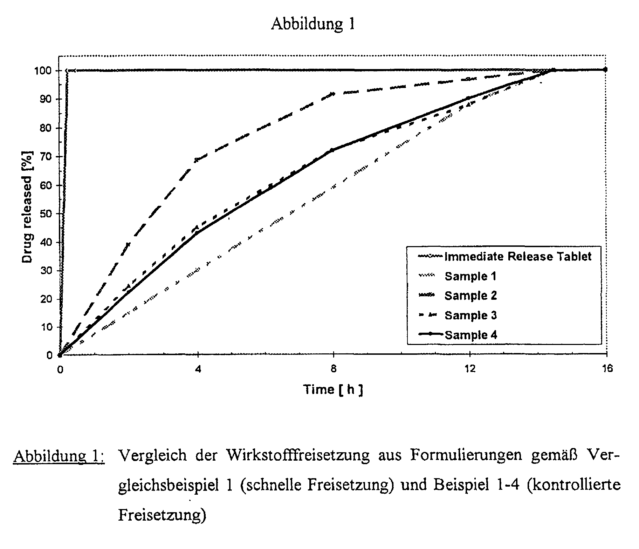

Ein Vergleich der Wirkstofffreisetzung (erhalten gemäß oben beschriebenen USP XXIII) der Formulierungen gemäß Vergleichsbeispiel 1 und Beispiel 1-4 ist in Abbildung 1 dargestellt.

**Beispiel 5**

(Diffusionspellets):

Zur Herstellung von Diffusionspellets werden 436 g der Verbindung II, 17,5 g Polyvinylpyrrolidon 25, 110 g Hydroxypropylmethylcellulose, 18 g Polyethylenglycol 4000, 220 g Ethylcellulose und 21 g Triethylcitrat zum Herstellen und Beschichten der Pellets in einer Wirbelschichtanlage eingesetzt. Die Pellets werden in Kapseln abgefüllt.

**Beispiel 6**

(Osmotisches Freisetzungssystem):

724,6 g Verbindung II, 182,5 g Kochsalz und 82,9 g mikrokristalline Cellulose werden granuliert, das Granulat mit 10 g Magnesiumstearat gemischt und diese Mischung zu Tabletten (Format 5,5 r 9) verpresst. Die Tabletten werden mit 49,8 g einer Mischung aus Celluloseacetat, Polyethylenglykol 3350 und Glycerol in acetonischer Lösung lackiert. Die Tabletten werden auf geeignete Weise angebohrt.

**Patentansprüche**

1. Arzneimittelformulierung mit kontrollierter Wirkstoff-Freisetzung, die 1-Cyclopropyl-7-([S,S]-2,8-diazabicyclo [4.3.0]non-8-yl)-6-fluor-1,4-dihydro-8-methoxy-4-oxo-3-chinoloncarbonsäure oder pharmazeutisch verträgliche Salze und/oder Hydrate davon umfaßt, und die eine mittlere Freisetzung zwischen 80 % in 2 Stunden und 80 % in 16 Stunden und eine initiale Freisetzung von weniger als 60 % des Wirkstoffs in der ersten Stunde der Freisetzung aufweist.

2. Arzneimittelformulierung nach Anspruch 1 mit einer mittleren Freisetzung zwischen 80 % in 4 Stunden und 80 % in 14 Stunden.

3. Arzneimittelformulierung nach Anspruch 1 oder 2 mit einer mittleren Freisetzung zwischen 80 % in 7 Stunden und 80 % in 13 Stunden und einer initialen Freisetzung von weniger als 55 % des Wirkstoffs in der ersten Stunde der Freisetzung.

4. Arzneimittelformulierung nach Anspruch 1 oder 2, worin die initiale Freisetzung in der ersten Stunde der Freisetzung zwischen 30 und 60 % des Wirkstoffs beträgt.

5. Arzneimittelformulierung nach irgendeinem der Ansprüche 1 bis 3, worin die initiale Freisetzung in der ersten Stunde der Freisetzung zwischen 0 und 30 % des Wirkstoffs beträgt.

6. Arzneimittelformulierung nach irgendeinem der Ansprüche 1, 2, 3 oder 4, worin die mittlere Freisetzung zwischen 80 % in 8 Stunden und 80 % in 12 Stunden und die initiale Freisetzung zwischen 45 und 55 % des Wirkstoffs in der ersten Stunde der Freisetzung beträgt.

7. Arzneimittelformulierung nach irgendeinem der Ansprüche 1, 2, 3 oder 5, worin die mittlere Freisetzung zwischen 80 % in 8 Stunden und 80 % in 12 Stunden und die initiale Freisetzung zwischen 0 und 20 % des Wirkstoffs in der ersten Stunde der Freisetzung beträgt.

8. Arzneimittelformulierung nach irgendeinem der Ansprüche 1 bis 7 in der Form von diffusionskontrollierten Pellets.

9. Arzneimittelformulierung nach Anspruch 8, **dadurch gekennzeichnet, daß** sie aus mit einer Mischung aus Wirkstoff und Bindemittel überzogenen Neutralpellets besteht, die mit einer Diffusionsschicht überzogen sind.

10. Arzneimittelformulierung nach Anspruch 8, **dadurch gekennzeichnet, daß** sie aus einem wirkstoffhaltigen Kern, der mit einem Diffusionslack überzogen ist, besteht.

11. Arzneimittelformulierung nach irgendeinem der Ansprüche 8 bis 10, **dadurch gekennzeichnet, daß** die Diffusionsschicht oder der Diffusionslack ein filmbildendes Polymer und einen Weichmacher enthält.

12. Arzneimittelformulierung nach irgendeinem der Ansprüche 8 bis 11, **dadurch gekennzeichnet, daß** die Diffusionsschicht oder der Diffusionslack ein filmbildendes Polymer und einen Porenbildner enthält.

13. Arzneimittelformulierung nach irgendeinem der Ansprüche 8 bis 12, **dadurch gekennzeichnet, daß** sie Hydroxypropylmethylcellulose oder Polyvinylpyrrolidon als Bindemittel, Ethylcellulose als filmbildendes Polymer und einen Weichmacher enthält.

14. Arzneimittelformulierung nach irgendeinem der Ansprüche 1 bis 7, **dadurch gekennzeichnet, daß** sie den Wirkstoff in einer Matrix eines wasserquellbaren Polymers umfaßt.

15. Arzneimittelformulierung nach Anspruch 14, **dadurch gekennzeichnet, daß** es sich um eine Tablette handelt.

16. Arzneimittelformulierung nach Anspruch 14 oder 15, **dadurch gekennzeichnet, daß** es sich bei dem wasserquellbaren Polymer um Hydroxypropylmethylcellulose oder Hydroxypropylcellulose handelt.

17. Arzneimittelformulierung nach irgendeinem der Ansprüche 1 bis 7, **dadurch gekennzeichnet, daß** es sich um ein osmotisches Arzneimittelfreisetzungssystem handelt.

18. Arzneimittelformulierung nach Anspruch 17, bestehend aus:

a) einem Kern, der den Wirkstoff, gegebenenfalls ein hydrophiles polymeres Quellmittel und gegebenenfalls einen wasserlöslichen Stoff zur Induzierung der Osmose enthält,

b) einer Hülle aus einem für Wasser durchlässigen und für die Komponenten des wirkstoffhaltigen Kerns undurchlässigen Material,

c) einer Öffnung durch die Hülle b) für den Transport der im Kern enthaltenen Bestandteile in die umgebende wäßrige Körperflüssigkeit.

19. Arzneimittelformulierung nach irgendeinem der Ansprüche 1 bis 18, **dadurch gekennzeichnet, daß** sie 1-Cyclopropyl-7-([S,S]-2,8-diazabicyclo[4.3.0]non-8-yl)-6-fluor-1,4-dihydro-8 -methoxy-4-oxo-3 -chinoloncarbonsäure-Hydrochlorid enthält.

20. Arzneimittelformulierung nach irgendeinem der Ansprüche 1 bis 19, **dadurch gekennzeichnet, daß** sie 1-Cyclopropyl-7-([S,S]-2,8-diazabicyclo[4.3.0]non-8-yl)-6-fluor-1,4-dihydro-8-methoxy-4-oxo-3-chinoloncarbonsäure-Hydrochlorid-Monohydrat enthält.

21. Arzneimittelformulierung nach irgendeinem der Ansprüche 1 bis 20, **dadurch gekennzeichnet, daß** sie bezogen auf die 1-Cyclopropyl-7-([S,S]-2,8-diazabicyclo[4.3.0]non-8-yl)-6-fluor-1,4-dihydro-8-methoxy-4-oxo-3-chinoloncarbonsäure 200 bis 800 mg des Wirkstoffs enthält.

22. Arzneimittelformulierung nach Anspruch 21, **dadurch gekennzeichnet, daß** sie bezogen auf die 1-Cyclopropyl-7-([S,S]-2,8-diazabicyclo[4.3.0]non-8-yl)-6-fluor-1,4-dihydro-8-methoxy-4-oxo-3-chinoloncarbonsäure 400 bis 600 mg des Wirkstoffs enthält.

**Claims**

1. Drug formulations having controlled release of active compound, which comprises 1-cyclopropyl-7-([S,S]-2,8-diazabicyclo[4.3.0]non-8-yl)-6-fluoro-1,4-dihydro-8-methoxy-4-oxo-3-quinolonecarboxylic acid or pharmaceutically tolerable salts and/or hydrates thereof and which has an average release between 80% in 2 hours and 80% in 16 hours and an initial release of less than 60% of the active compound in the first hour of release.

2. Drug formulation according to Claim 1, having an average release between 80% in 4 hours and 80% in 14 hours.

3. Drug formulation according to Claim 1 or 2, having an average release between 80% in 7 hours and 80% in 13 hours and an initial release of less than 55% of the active compound in the first hour of release.

4. Drug formulation according to Claim 1 or 2, in which the initial release in the first hour of release is between 30 and 60% of the active compound.

5. Drug formulation according to any of Claims 1 to 3, in which the initial release in the first hour of release is between 0 and 30% of the active compound.

6. Drug formulation according to any of Claims 1, 2, 3 or 4, in which the average release is between 80% in 8 hours and 80% in 12 hours and the initial release is between 45 and 55% of the active compound in the first hour of release.

7. Drug formulation according to any of Claims 1, 2, 3 or 5, in which the average release is between 80% in 8 hours and 80% in 12 hours and the initial release is between 0 and 20% of the active compound in the first hour of release.

8. Drug formulation according to any of Claims 1 to 7 in the form of diffusion-controlled pellets.

9. Drug formulation according to Claim 8, **characterized in that** it comprises neutral pellets coated with a mixture of active compound and binder which are coated with a diffusion layer.

10. Drug formulation according to Claim 8, **characterized in that** it comprises an active-compound-containing core which is coated with a diffusion coat.

11. Drug formulation according to any of Claims 8 to 10, **characterized in that** the diffusion layer or the diffusion coat contains a film-forming polymer and a plasticizer.

12. Drug formulation according to any of Claims 8 to 11, **characterized in that** the diffusion layer or the diffusion coat contains a film-forming polymer and a pore former.

13. Drug formulation according to any of Claims 8 to 12, **characterized in that** it contains hydroxypropylmethylcellulose or polyvinylpyrrolidone as binder, ethylcellulose as film-forming polymer and a plasticizer.

14. Drug formulation according to any of Claims 1 to 7, **characterized in that** it comprises the active compound in a matrix of a water-swellable polymer.

15. Drug formulation according to Claim 14, **characterized in that** it is a tablet.

16. Drug formulation according to Claim 14 or 15, **characterized in that** the water-swellable polymer is hydroxypropylmethylcellulose or hydroxypropylcellulose.

17. Drug formulation according to any of Claims 1 to 7, **characterized in that** it is an osmotic drug release system.

18. Drug formulation according to Claim 17, comprising:

   a) a core which contains the active compound, optionally a hydrophilic polymeric swelling agent and optionally a water-soluble substance for inducing osmosis,

   b) a shell of a material which is water-permeable and impermeable for the components of the active-compound-containing core,

c) an opening through the shell b) for the transport of the components contained in the core into the surrounding aqueous body liquid.

19. Drug formulation according to any of Claims 1 to 18, **characterized in that** it contains 1-cyclopropyl-7-([S,S]-2,8-diazabicyclo[4.3.0]non-8-yl)-6-fluoro-1,4-dihydro-8-methoxy-4-oxo-3-quinolonecarboxylic acid hydrochloride.

20. Drug formulation according to any of Claims 1 to 19, **characterized in that** it contains 1-cyclopropyl-7-([S,S]-2,8-diazabicyclo[4.3.0]non-8-yl)-6-fluoro-1,4-dihydro-8-methoxy-4-oxo-3-quinolonecarboxylic acid hydrochloride monohydrate.

21. Drug formulation according to any of Claims 1 to 20, **characterized *in* that** it contains 200 to 800 mg of the active compound, based on 1-cyclopropyl-7-([S,S]-2,8-diazabicyclo[4.3.0]non-8-yl)-6-fluoro-1,4-dihydro-8-methoxy-4-oxo-3-quinolonecarboxylic acid.

22. Drug formulation according to Claim 21, **characterized in that** it contains 400 to 600 mg of active compound, based on 1-cyclopropyl-7-([S,S]-2,8-diazabicyclo[4.3.0]non-8-yl)-6-fluoro-1,4-dihydro-8-methoxy-4-oxo-3-quinolonecarboxylic acid.

## Revendications

1. Formulation médicamenteuse avec libération contrôlée du principe actif, qui comprend l'acide 1-cyclopropyl-7-([S,S]-2, 8-diazabicyclo[4.3.0]non-8-yl)-6-fluoro-1,4-dihydro-8-méthoxy-4-oxo-3-quinolonecarboxylique ou ses sels pharmaceutiquement compatibles et/ou ses hydrates et qui présente une libération moyenne allant de 80% en 2 heures à 80% en 16 heures et une libération initiale inférieure à 60% du principe actif dans la première heure de libération.

2. Formulation médicamenteuse suivant la revendication 1, avec une libération moyenne allant de 80% en 4 heures à 80% en 14 heures.

3. Formulation médicamenteuse suivant la revendication 1 ou 2, avec une libération moyenne allant de 80% en 7 heures à 80% en 13 heures et une libération initiale inférieure à 55% du principe actif dans la première heure de libération.

4. Formulation médicamenteuse suivant la revendication 1 ou 2, pour laquelle la libération initiale au cours de la première heure de libération se situe dans l'intervalle allant de 30 à 60% du principe actif.

5. Formulation médicamenteuse suivant l'une quelconque des revendications 1 à 3, pour laquelle la libération initiale au cours de la première heure de libération se situe dans l'intervalle allant de 0 à 30% du principe actif.

6. Formulation médicamenteuse suivant l'une quelconque des revendications 1, 2, 3 ou 4, pour laquelle la libération moyenne se situe dans l'intervalle allant de 80% en 8 heures à 80% en 12 heures et la libération initiale se situe dans l'intervalle allant de 45 à 55% du principe actif dans la première heure de libération.

7. Formulation médicamenteuse suivant l'une quelconque des revendications 1, 2, 3 ou 5, pour laquelle la libération moyenne se situe dans l'intervalle allant de 80% en 8 heures à 80% en 12 heures et la libération initiale se situe dans l'intervalle allant de 0 à 20% du principe actif dans la première heure de libération.

8. Formulation médicamenteuse suivant l'une quelconque des revendications 1 à 7, sous la forme de pellets à diffusion contrôlée.

9. Formulation médicamenteuse suivant la revendication 8, **caractérisée en ce qu'**elle consiste en des pellets neutres revêtus d'un mélange du principe actif et d'un liant, lesquels sont revêtus d'une couche de diffusion.

10. Formulation médicamenteuse suivant la revendication 8, **caractérisée en ce qu'**elle consiste en un coeur contenant le principe actif, qui est revêtu d'un vernis de diffusion.

11. Formulation médicamenteuse suivant l'une quelconque des revendications 8 à 10, **caractérisée en ce que** la couche de diffusion ou le vernis de diffusion contient un polymère filmogène et un plastifiant.

12. Formulation médicamenteuse suivant l'une quelconque des revendications 8 à 11, **caractérisée en ce que** la couche de diffusion ou le vernis de diffusion contient un polymère filmogène et un agent porogène.

13. Formulation médicamenteuse suivant l'une quelconque des revendications 8 à 12, **caractérisée en ce qu'**elle contient l'hydroxypropylméthylcellulose ou la polyvinylpyrrolidone comme liant, l'éthylcellulose comme polymère filmogène et un plastifiant.

14. Formulation médicamenteuse suivant l'une quelconque des revendications 1 à 7, **caractérisée en ce qu'**elle comprend le principe actif dans une matrice d'un polymère gonflable dans l'eau.

15. Formulation médicamenteuse suivant la revendication 14, **caractérisée en ce qu'**il s'agit d'un comprimé.

16. Formulation médicamenteuse suivant la revendication 14 ou 15, **caractérisée en ce que** le polymère gonflable dans l'eau consiste en l'hydroxypropylméthylcellulose ou l'hydroxypropylcellulose.

17. Formulation médicamenteuse suivant l'une quelconque des revendications 1 à 7, **caractérisée en ce qu'**il s'agit d'un système osmotique de libération du médicament.

18. Formulation médicamenteuse suivant la revendication 17, consistant en :

a) un coeur qui contient le principe actif, le cas échéant un agent gonflant de type polymère hydrophile et le cas échéant, une substance soluble dans l'eau pour induire l'osmose ;
b) une enveloppe perméable à l'eau et imperméable pour les composants du coeur contenant le principe actif, et
c) une ouverture dans l'enveloppe b) pour le transport des constituants contenus dans le coeur dans le liquide corporel aqueux environnant.

19. Formulation médicamenteuse suivant l'une quelconque des revendications 1 à 18, **caractérisée en ce qu'**elle contient le chlorhydrate de l'acide 1-cyclopropyl-7-([S, S]-2,8-diazabicyclo[4.3.0]non-8-yl)-6-fluoro-1,4-dihydro-8-méthoxy-4-oxo-3-quinolonecarboxylique.

20. Formulation médicamenteuse suivant l'une quelconque des revendications 1 à 19, **caractérisée en ce qu'**elle contient le chlorhydrate monohydraté de l'acide 1-cyclopropyl-7-([S,S]-2,8-diazabicyclo[4.3.0]-non-8-yl)-6-fluoro-1,4-dihydro-8-méthoxy-4-oxo-3-quinolonecarboxylique.

21. Formulation médicamenteuse suivant l'une quelconque des revendications 1 à 20, **caractérisée en ce qu'**elle contient, sur base de l'acide 1-cyclopropyl-7-([S,S]-2,8-diazabicyclo[4.3.0]non-8-yl) -6-fluoro-1,4-dihydro-8-méthoxy-4-oxo-3-quinolonecarboxylique, 200 à 800 mg du principe actif.

22. Formulation médicamenteuse suivant la revendication 21, **caractérisée en ce qu'**elle contient, sur base de l'acide 1-cyclopropyl-7-([S,S]-2,8-diazabicyclo[4.3.0]non-8-yl)-6-fluoro-1,4-dihydro-8-méthoxy-4-oxo-3-quinolonecarboxylique, 400 à 600 mg du principe actif.

Abbildung 1

Abbildung 1: Vergleich der Wirkstofffreisetzung aus Formulierungen gemäß Vergleichsbeispiel 1 (schnelle Freisetzung) und Beispiel 1-4 (kontrollierte Freisetzung)